# EUROPEAN PATENT APPLICATION

(11) **EP 1 917 980 A1**
(43) Date of publication of application: **07.05.2008**
(21) Application number: 06076951.0
(22) Date of filing: 31.10.2006
(51) Int. Cl.: A61K 47/48, A61K 38/19, A61K 38/20, A61P 9/10

(54) **Use of a fusion protein between a cytokine and an antibody targeting the ED-B fibronectin domain for the treatment of atherosclerosis**

(71) Applicant: Bayer Schering Pharma Aktiengesellschaft, 13353 Berlin (DE)
(72) Inventor: Menrad, Andreas, Dr., Ely Cambs CB6 2HP (GB); Menssen, Hans Dietrich, Dr., 10585 Berlin (DE); Graf, Kristof, Prof., Dr., 14532 Kleinmachnow (DE)

(57) **Abstract**

The present invention relates to the use of a fusion protein comprising an antibody part which specifically recognises the oncofetal extra domain B (ED-B) of fibronectin, an effector part and optionally one or more fusion protein linker(s) and/or antibody linker(s), for the manufacturing of medicaments for the treatment of atherosclerosis

## Description

The present invention relates to the use of a fusion protein comprising an antibody part which specifically recognises the extra domain B (ED-B) of fibronectin, an effector part and optionally one or more fusion protein linker(s) and/or antibody linker(s), for the manufacturing of medicaments for the treatment of atherosclerosis.

### State of the Art

**Atherosclerosis** is known as a chronic inflammatory lipid storage disease of large and medium-sized arteries complicated by cardiovascular events. These are commonly the result of sudden arterial thrombosis in the heart, brain, legs, and other organs. Pathologic intimal thickening as a result of phospholipids and cholesterol deposition constitutes the earliest detectable atherosclerotic change which is followed by macrophage and CD4+ and CD8+ T cell invasion (Virami R, et al., Arterioscler, Thromb Vasc Biol 2005, 25:2054-61; Xu QB, et al., Clin Immunol Immunpathol, 1990, 56: 344-359). Subsequently, this process leads to proliferation of the *vasa vasorum* which perfuses the atherosclerotic plaques (Rose R. N Engl J Med., 1999, 340:115-126; Wilson SH, et al., Circulation., 2002, 105:415-418). Rupture of the *vasa vasorum* resulting in intraplaque haemorrhage is an important process in the progression of asymptomatic plaques into high-risk unstable lesions (Kolodgie FD, et al., N Engl J Med., 2003, 349:2316-2325). Intraplaque heamorrhage and plaque rupture are associated with an increased density of mircovessels (Fleiner M, et al., Circulation 2004, 110, 2843-2850). Plaque rupture is the principal cause of luminal thrombosis in acute coronary artery syndromes occurring in 75% of patients dying of acute myocardial infarction (Davies MJ, et al., N Engl J Med, 1984, 310: 1137-1140).Methods for the effective imaging of said atherosclerotic plaques and methods of treatment thereof are of considerable interest.

The prevention of **coronary artery disease** (CAD) or atherosclerosis in general involves therapeutic lifestyle changes such as smoking cessation, diet, weight reduction and exercise. In patients with established CAD or atherosclerosis in other vascular beds, or in patients at high risk of developing CAD, lowering serum total and low-density lipoprotein cholesterol (LDL-C) has been associated with a reduction in cardiovascular morbidity and mortality, and total mortality. Therapy with 3-hydroxy-3-methylglutaryl coenzyme A reductase inhibitors (such as atorvastatin, Lipitor®; pravastatin, Pravachol®; simvastatin, Zocor)) has had a major impact on preventive cardiology. Clinical trials have consistently shown that the reduction in serum cholesterol correlates with a decrease in major cardiovascular events, irrespective of the method used to reduce cholesterol. However, there is little trial-based evidence to support the early clinical data of these agents in patients with acute coronary syndromes. Usually, these patients are treated with platelet inhibitors (e.g. Aspirin® or clopidogrel, Plavix®), anticoagulants (sc heparin injections), and vaso-dilators (e.g. nitroglycerine, calcium antagonists). These systemic therapies may transiently reduce clinical symptoms in CAD patients, but they are not known for reducing the plaque size, improve or stabilize the plaque morphology, or having a striking early impact on progressive atherosclerosis. For manifest and or clinically symptomatic CAD, the current therapy of choice includes coronary balloon angioplasty and coronary artery bypass graft surgery (Braunwald E, et al., in Harrison's Principle of Internal Medicine, most recent edition (2005), McGraw-Hill).

Although these invasive therapies may result in relief of acute or subacute symptoms, since local in nature, they have no major impact on the underlying progressive atherosclerotic disease.

One of the most selective markers associated with angiogenesis known so far represents the extra **domain B (ED-B) of fibronectin (FN)**. FNs are high molecular-mass extracellular matrix (ECM) components abundantly expressed in a range of healthy tissues and body fluids. Various different FN isoforms can be generated due to alternative splicing at the level of the primary transcript. The ED-B, a small domain of 91 amino acids, which is identical in sequence in mouse, rat and man, is usually absent in both plasma and tissue-fibronectin, except for some blood vessels of the regenerating endometrium and the ovaries. (Alessi P. et al., Biochim. Biophys. Acta, 2004, 1654 : 39-49; Viti F. et al., Cancer Res., 1999, 59 : 347-352). However, it may become inserted into the fibronectin molecule during active tissue remodeling associated with neo-angiogenesis, thereby specifically accumulating around neovascular structures, such as around the neo-vasculature in atherosclerotic plaques. Thus, the ED-B domain of fibronectin represents a target for molecular intervention (Zardi et al., EMBO J., 1987, 6 : 2337-2342, Carnemolla et al., J. Cell Biol., 1989, 108 : 1139-1148, Castellani et al., Int. J. Cancer, 1994, 59 : 612-618).

In this context, molecules capable of selectively targeting markers of angiogenesis create clinical opportunities for the diagnosis and therapy of diseases characterised by vascular proliferation, such as rheumatoid arthritis, diabetic retinopathy and age-related macular degeneration (O'Reilly et al., Nat. Med., 1996, 2 : 689, O'Reilly et al., Cell, 1997, 88 : 277, Friedlander et al., Science, 1995, 270 : 1500, Pasqualini et al., Nat. Biotechnol., 1997, 15 : 542, Huang et al., Science, 1997, 275 : 547, Kim et al., Nature, 1993, 362 : 841, Schmidt-Erfurth et al., Br. J. Cancer, 1997, 75 : 54).

Recently, a number of good quality **antibodies specific for the ED-B of fibronectin** have been generated. In particular, the human single chain Fv antibody fragment L19, which displays a picomolar binding affinity for ED-B, has been verified to selectively target newly formed tumor blood vessels , e.g. in experimental tumor models (Viti F. et al., Cancer Res., 1999, 59 : 347-352; Tarli L, et al., Blood, 1999, 94: 192-198) and tumor lesions in patients with solid cancers (Santimaria M. et al., Clin. Cancer Res., 2003, 9 : 571-579).

Cytokines are a group of proteinaceous signaling compounds that are used extensively for inter-cell communication. Apart from their importance in the development and functioning of the immune system, cytokines also play a major role in a large number of immunological, inflammatory and infectious diseases. It has been shown in the past that cytokines can be very potent compounds for the treatment of disorders in the human and animal body. Interferon-alpha is active as a monotherapy against chronic myeloid leukemia (CML) and hairy cell leukemia and can induce complete long-lasting remissions in some patients (Kamtarjian HM, et al., Blood 2006, 108:1835-1840; Baker PK, et al., Blood 2002, 100:647-653; Damasio EE, et al., Eur J Haematol 2000, 64: 42-52). Pegylated Interferon-alpha is widely used as an active treatment, alone or in combination with lamivudine or adefovir, against chronic hepatitis B, C, and D (Wursthorn K, et al., Hepatology 2006, 44: 675-684; Desmond, CP, et al., J Viral Hepatitis 2006, 13: 311-315; Niro GA, et al., Hepatology 2006, 44:713-720). Interferon beta is widely used as a treatment for multiple sclerosis (Kappos L, et al., Neurology 2006, 67:944-953). Tumour necrosis factor alpha (TNFα) is approved in combination with melphalan as a limb-sparing therapy for sarcoma patients in the context of isolated limb perfusion (ILP; Grunhagen D, et al., Cancer 2006, 106:1776-84). It is also successfully used in the same setting for melanoma patients (Lejeune F, et al., Cancer Immunity, 2006, 6: 1-17).

Particularly, **interleukin-2 (IL2)** has been characterized as one of the most potent cytokines, especially in anti-tumor experiments. It exhibits panoply of immune regulatory effects, including the stimulation of various anti-tumor effector cells. (Rosenberg S.A., J. Intern. Med., 2001, 250 : 462-475). However, despite being approved for the clinical treatment of metastatic renal cell carcinoma, systemically applied IL2 has not been proven as successful as one had hoped. Therapeutic efficacy of systemically applied IL2 is thwarted by its serious, potentially life-threatening side effects (e.g. orthostatic hypotension, vascular leak syndrome and profound malaise) that limit dose escalation and prevent the administration of a curative dose. (Bubenik J. et al., Cancer Immunol. Immunother. 2000, 49 : 116-122; Baluna R. et al., Proc. Natl. Acad. Sci. USA, 1999 , 96 : 3957-3962). Additionally, the rapid degradation or elimination of IL2 delivered systemically further decreases its effectiveness. On the other hand, local administration of IL2 has been more successful and has resulted in the control of malignant effusions and the generation of significant remission of established tumour lesions. (Bubenik J. et al., Cancer Immunol. Immunother., 2000, 49 : 116-122; Den Otter W. et al., J. Urol., 1998, 159 : 1183-1186; Baselmans A.H. et al., Cancer Immunol. Immunother., 2002, 51 : 492-498; Krastev Z. et al., Hepatogastroenterology, 2003, 50 : 1647-1649, Radny P, et al., Br J Cancer 2003, 89:1620-1626).

Matter *et al.* have for example described imaging methods for displaying atherosclerotic plaques in ApoE-/- mice (atherosclerosis model) using an fibronectin ED-B-specific antibody, which has been labelled with a radioactive or infrared-sensitive marker (cf. Matter C.M. et al., Circulation Research, 2004, 24 : 1225-1233). However, the vague therapeutic outlook proposed by Matter *et al.,* according to which human monoclonal antibody L19 fused with interleukin-12 (IL12), among other L19-based fusion proteins, might eventually be therapeutically effective against said atherosclerotic plaques, does not match up with the scientific findings of others. (cf. Davenport P. et al., Am. J. Pathology, 2003, 20 : 264-269; Zhao L. et al., JBC, 2002, 277 : 35350-35356; Lee T.S. et al., Atheroscler. Throm. Vasc. Biol., 1999, 19 : 737-742; Zhou R.H. et al., J. Atheroscler. Thromb., 2001, 8 : 30-32; Hauer A.D. et al., Circulation, 2005, 16 : 1054-1062). Accordingly, presence of IL12 in atherosclerotic plaques has been identified as a key inducer of a type 1 T-helper cell cytokine pattern, which is thought - in striking contrast to the suggestion of Matter *et al*., - to contribute to the development of atherosclerosis, rather than to prevent or treat it. In particular, Davenport *et al.* have shown that IL12-knockout in ApoE-/- mice leads to a significant reduction in plaque formation. This result is supported Zhao *et al*., who used targeted gene disruption or overexpression of 12/15-lipoxygenase in mice (background of apolipoprotein E or low density lipoprotein-receptor deficiency) and found a 50% decrease in aortic lesions at 8 months in mice on chow diet (no cholesterol difference). In the cultured macrophages of these mice they discovered a remarkable 75-90% decrease in IL12 production. Lee *et al*. found IL12 expression in macrophages of aortic plaques of apo-E-deficient mice, and that daily applications of IL12 to these mice accelerated atherosclerosis. Similarly, Zhou R.H. *et al.* have found elevated serum IL-12 levels in patients with acute myocardial infarction but not in patients with unstable angina pectoris, suggesting that IL12 is involved in the progression of CAD. Finally, these findings prompted Hauer *et al*. to design a vaccination technique in mice that fully blocks the biologic action of IL12. Vaccination of LDL receptor-deficient mice (LDLr-/-; another atherosclerosis animal model) against the biologic activity of IL12 resulted in a significant 68.5% reduction of artherogenesis.

The publication Subramanya Upadhya et al. (2004) Atherogenic effect of Interleukin-2 and antiatherogenic effect of Interleukin-2 antibody in Apo-E-Deficient Mice, Angiology, Vol. 55 No. 3, pages 289 - 294 demonstrate a pro - atherosclerotic effect caused by interleukin-2 enhancing atherosclerosis. Further anti - IL-2 antibodies have a protective effect against atherosclerosis.

In view of the above, an urgent need exists for compositions and/or medicaments which can be used in the specific treatment of said atherosclerotic plaques, and according to the scientific data stated, IL12 or targeted IL12 obviously cannot be considered a possible solution to this therapeutic problem.

### Problem and Solution

Thus, the technical problem underlying the present invention is to overcome the above-mentioned problems by providing novel medicaments for the specific treatment of atherosclerotic plaques in humans and animals and other diseases connected thereto.

The solution to the above technical problem is achieved by providing the embodiments characterized in the claims.

In particular, there is provided the use of a fusion protein comprising at least one targeting part and at least one effector part, wherein the targeting part specifically recognises the extra domain B (ED-B) of fibronectin and wherein the effector part is a cytokine or a biologically active fragment thereof, in the manufacture of a medicament for the treatment of atherosclerosis.

The term "fusion protein" used herein relates to an artificial proteinaceous construct and means a protein comprising at least two different amino acid sequences which are defined by their origin and/or by special functions. Moreover, the term fusion protein according to the present invention does further include such fusion proteins which also contain non-protein molecules such as nucleic acids, sugars, or markers for radioactive or fluorescent labelling.

Further, the term "targeting part" used according to the present invention means any molecule or group of molecules which selectively binds to at least a portion of a desired target molecule, such as a receptor, an antigen or fragments thereof. Examples of said targeting part include hormones, neurotransmitters, antibodies and fragments thereof.
As used herein, the expressions "fragment" or "biologically active fragment" mean a part or a combination of parts of a molecule or group of molecules, as long as the desired effects, such as targeting activity or biological and pharmaceutical activity, are maintained.

According to one embodiment of the present invention, in the use as defined above the targeting part is an antibody or a fragment thereof.

The term "antibody" used in the present invention is not specifically limited and includes for example antibodies, antibody fragments, native antibodies, monoclonal antibodies, monoclonal antibodies isolated from phage antibody libraries, chimeric antibodies, humanised antibodies, an antagonist "which binds" an antigen of interest, antibody fragments isolated from antibody phage libraries and human antibodies.

Said term "antibody" is further used in the broadest sense and specifically covers intact monoclonal antibodies, polyclonal antibodies, multispecific antibodies (e.g. bispecific antibodies) formed from at least two intact antibodies, and antibody fragments thereof as long as they exhibit the desired biological targeting activity.

The term "antibody fragments" used herein comprises a portion of an intact antibody, preferably comprising the antigen-binding or variable region thereof. Examples of such antibody fragments include Fab, Fab', F(ab')2 and Fv fragments, diabodies, minibodies, linear antibodies, single-chain antibody molecules, "scFv", and multispecific antibodies formed from antibody fragments.

According to one embodiment of the use according as defined above, the targeting part is the human recombinant antibody L19 or a fragment thereof.

According to a specific example of the above-defined use of the present invention, the heavy chain of the antibody has a sequence according to SEQ ID NO: 1 and/or the light chain of said antibody has a sequence according to SEQ ID NO: 2.

According to another embodiment, in the use as defined above, the heavy and the light chain of the antibody are connected by an antibody linker.

The term "antibody linker" as used herein is not especially restricted and may be any antibody linker known in the art, such as an amino acid, a peptide, or an aliphatic or aromatic organic molecule. Examples of such linkers are described in EP 0 573 551, EP 0 623 679 and EP 0 318 554.

In a specific example of the above-defined use, the antibody linker has a sequence according to SEQ ID NO: 3.

According to the present invention, the term "effector part" is not specifically restricted and means any cytokine known in the art, excluding interleukin-12 (IL12). Examples of such cytokines are interleukins (IL) such as IL1α and IL1β, IL2 to IL11 or IL 13 to IL22, as well as interferons (IFN), such as IFN-α, IFN-β or IFN-γ, and Tumour Necrosis Factor alpha (TNFα).

In a further embodiment of the above-defined use present invention, the cytokine is an interleukin or a biologically active fragment or derivatives thereof.

According to a specific embodiment of the present invention, the cytokine in the above-defined use is interleukin-2 (IL2) or a fragment or derivative thereof or a Tumour Necrosis Factor alpha (TNFα) or a fragment or derivative thereof.

According to one example of the present invention, the interleukin-2 has a sequence according to SEQ ID NO: 4 or the TNF alpha sequence described in SEQ ID NO: 6.

According to a preferred embodiment of the present invention, the fusion protein contains L19-IL2 and has a sequence according to the addition of the sequences SEQ ID NO: 1, SEQ ID NO: 3, SEQ ID NO: 2, SEQ ID NO: 5 and SEQ ID NO: 4. (in the direction from the N terminal to the C terminal)
The fusion protein L19 - TNF alpha comprises the following succession of elements: SEQ ID NO: 1, SEQ ID NO: 3, SEQ ID NO: 2, SEQ ID NO: 5 and SEQ ID NO: 6. (in the direction from the N terminal to the C terminal)

In one embodiment of the present invention, the fusion protein in the above-defined use has (a) an N-terminal targeting part and a C-terminal effector part, or (b) an N-terminal effector part and a C-terminal targeting part.

According to another embodiment of the present invention, the targeting part specifically binds to the oncofetal ED-B of fibronectin.

In another embodiment of the above-defined use, the targeting part and the effector part are connected by a fusion protein linker. A preferred embodiment of this fusion protein linker is described in SEQ ID NO 5.

The term "fusion protein tinker" used herein is not specifically restricted and may include any linker usable to connect the targeting part and the effector part according to the present invention, such as an amino acid, a peptide or an aliphatic or aromatic organic molecule. Specific examples of fusion protein linkers are described in EP 0 573 551, EP 0 623 679 and EP 0 318 554.

Another aspect of the present invention relates to the method of treating atherosclerotic plaques and the diseases connected thereto in a patient, comprising the step of administering a fusion protein according to the present invention to said patient.

The fusion protein according to the present invention may be administered typically in combination with one or more auxiliary agents, such as pharmaceutically acceptable carriers, buffers or salts.

The route of administration of the composition of the present invention does not exhibit a specific limitation and can be, for example, subcutaneous or intravenous. The term "patient" as used in the present invention includes mammals, particularly humans.

The fusion protein according to the present invention may be administered in an amount of 5 to 60 Mio IU IL2 equivalent (corresponding to 0.835 to 10.02 mg fusion protein) per application and human patients. Treatment might be given in a repeated fashion parenterally either iv or sc. (e. g. on day 1, 3, and 5, repeat on day 22 or possibly daily). Different application schemes might be necessary to obtain full clinical benefit.

The figures show:
- Fig. 1 shows: the schematic course of the L19-IL2 fusion protein experiments in ApoE(-/-) mice.

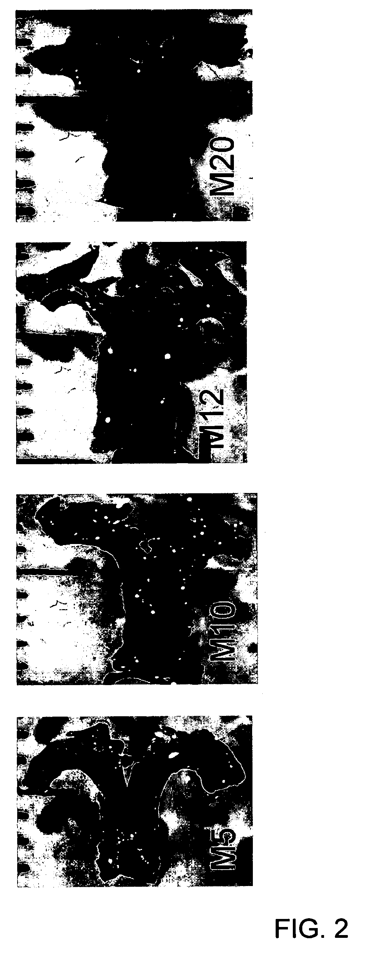

- Fig. 2 shows: photographs of thoracic aortas prepared from ApoE(-/-) mice. The dark regions indicate fatty lesions visualized using Sudan dyes.
These lesions correspond to atherosclerotic plaques.
- Fig. 3 shows: a diagram on the effect of L19-IL2 fusion protein on atherosclerotic plaque formation in thoracic aortas of ApoE(-/-) mice at an age of 5 months and fed with a high fat diet.

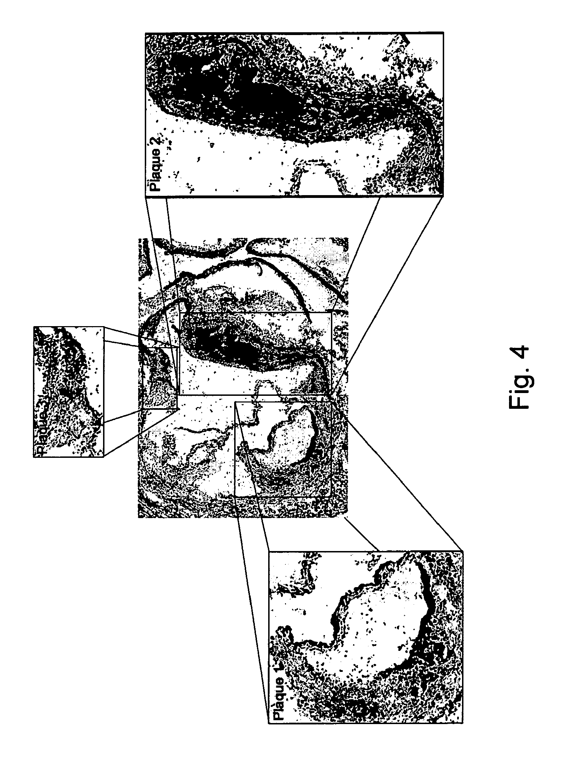

- Fig. 4 shows: the photographs of histo-morphology of ED-B in atherosclerotic plaques of the aortic root of 6-months-old apoE (-/-) mice fed with normal chow (normal diet). Dark areas indicate high accumulation of the ED-B of fibronectin binder especially around the vasa vasorum of the plaques and in the endothelial linings of the plaque cap.

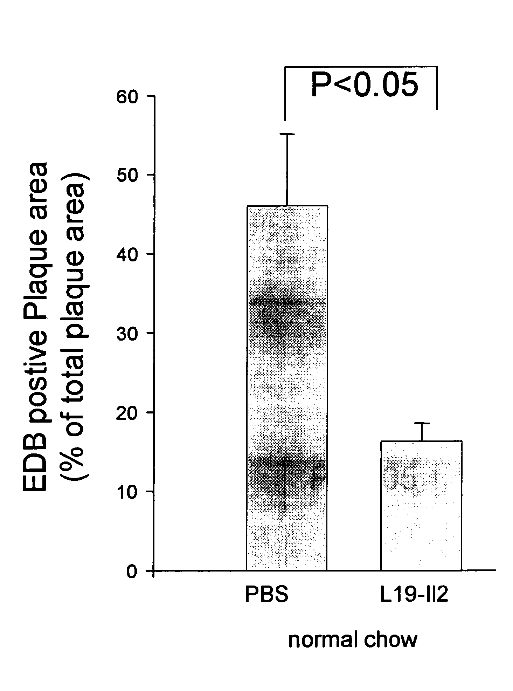

- Fig. 5 shows: the effect of L19-IL2 on formation of ED-B positive plaque area in the aortic root of ApoE(-/-) mice (6 month old) fed with normal fat diet.

The present invention provides a fusion protein which comprises at least one targeting part and at least one effector part, wherein the targeting part specifically recognises the oncofetal extra domain B (ED-B) of fibronectin, in the manufacture of a medicament for the treatment of atherosclerosis. Since ED-B expression can only be found in very few tissues and/or locations in the human and animal body, such as in atherosclerotic plaques, and since it is not expressed in the majority of healthy mature human organs, the use according to the present invention advantageously allows for the production of highly specific medicaments which target these tissues and/or locations. Thus, through the use of the fusion protein according to the present invention, the obtained medicaments enable the transport of the effector part to the desired tissue or location. Due to the surprisingly high specificity of the fusion protein for the ED-B of fibronectin, a medicament is provided that allows, by use of a suitable effector part, such as the cytokine IL2 or TNFα, the direct and highly efficient treatment of the targeted tissue and/or location.

The targeted concentration of a cytokine such as IL2 in the atherosclerotic plaque microenvironment by conjugating it for example to the homodimeric scFv L19 antibody, specific for the ED-B of fibronectin, enhances the therapeutic index of the cytokine and at the same time diminishes its toxic side effects, thereby providing a valuable therapeutic tool for the treatment of atherosclerotic plaques and further diseases connected thereto.

### EXAMPLES

The present invention will be further illustrated in the following examples, without any limitation thereto.

### Example 1: Treatment of ApoE mice with fusion protein L19-IL2

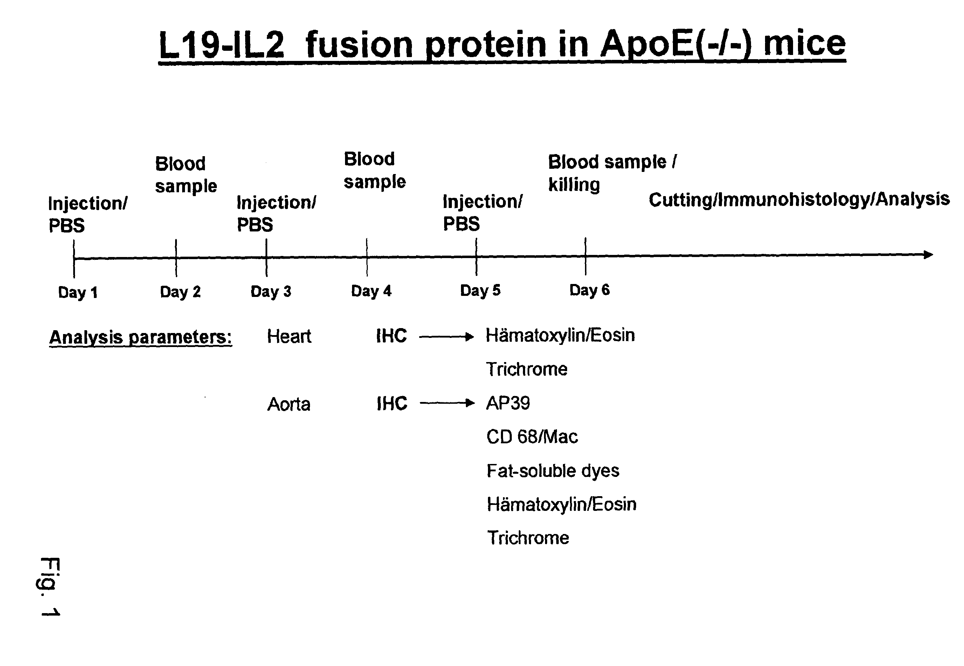

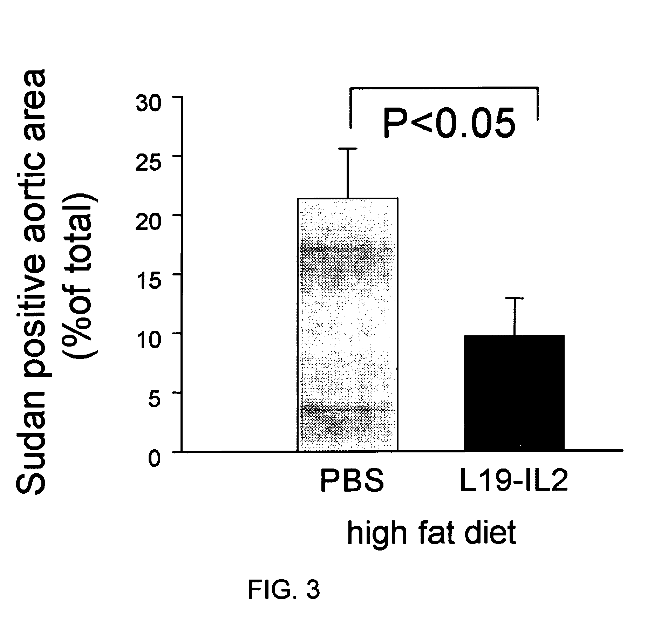

For evaluation of the progression of atherosclerosis, myocardial events and mortality 6 to 7 months old ApoE(-/-) mice are fed with normal chow and high fat diet. The fusion protein L19-IL2 is applied 3 times/week in steril phosphate buffered saline via i.v. injection at a dose of 10⁶ IU/kg IL2 equivalents. Blood was collected by retro-orbital bleeding. A diagram showing the experimental course is given in Figure 1.
The result of these experiments show that is was surprisingly found that the addition of L10-IL2 leads to reduced plaque formation (Figure 3)

### Example 2: Histology of the heart

For determining the impact of the fusion protein L19-IL2 on the histology of the heart, both hearts of L19-IL2-treated animals and of control animals are analysed for areas of infarction, infiltration of mononuclear cells and obliterated coronary arteries. For this purpose, tissue samples were snap-frozen in liquid nitrogen, embedded in OCT and stored at -80°C for immunohistochemistry. For this purpose, 5-10µM tissue sections were fixed and stained with the respective antibodies according to standard immunohistochemical procedures.

The histological experiments demonstrate that no significant differences between control and L19-IL2-treated animals occur.

### Example 3: Serum parameters

According to these serologic experiments both control- and L19-IL2 groups show no differences in troponin levels (a highly sensitive and specific indicator of myocardial infarction).

## Claims

1. Use of a fusion protein comprising at least one targeting part and at least one effector part, wherein the targeting part specifically recognises the extra domain B (ED-B) of fibronectin and wherein the effector part is a cytokine or a biologically active fragment thereof, in the manufacture of a medicament for the treatment of atherosclerosis.

2. The use according to claim 1, wherein the targeting part is an antibody or a biologically active fragment thereof.

3. The use according to claim 1 or 2, wherein the targeting part is the human recombinant antibody L19 or a biologically active fragment thereof.

4. The use according to claim 2 or 3, wherein the heavy chain of the antibody has a sequence according to SEQ ID NO: 1 and/or the light chain of said antibody has a sequence according to SEQ ID NO: 2.

5. The use according to any one of claims 2 to 4, wherein the heavy and the light chain of the antibody are connected by an antibody linker.

6. The use according to any one of claims 2 to 5, wherein the antibody linker has a sequence according to SEQ ID NO: 3.

7. The use according to any one of claims 1 to 6, wherein the cytokine is an interleukin or a biologically active fragment thereof.

8. The use according to claim 7, wherein the cytokine is interleukin-2 (IL2), or tumor necrosis factor-alpha (TNFα), or a biologically active fragment thereof.

9. The use according to claim 8, wherein the interleukin-2 and tumor necrosis factor-alpha (TNFα), have a sequence according to SEQ ID NO: 4 and

10. The use according to any one of the preceding claims, wherein the fusion protein has
(a) an N-terminal targeting part and a C-terminal effector part, or
(b) an N-terminal effector part and a C-terminal targeting part.

11. The use according to any one of the preceding claims, wherein the targeting part specifically binds to the oncofoetal ED-B of fibronectin.

12. The use according to any one of the preceding claims, wherein the targeting part and the effector part are connected by a fusion protein linker.

13. The use according to claim 12, wherein the fusion protein linker has a sequence according to SEQ ID NO: 5.


### Annex to the application documents - subsequently filed sequences listing

SEQUENCE LISTING

<110>  BAYER SCHERING PHARMA AKTIENGESELLSCHAFT

<120>  Use of a fusion protein targeting the ED-B fibronectin domain for
treatment of artherosclerosis

<130>  53471AEP

<140>  EP 06076951.0

<141>  2006-10-31

<160>  6

<170>  PatentIn version 3.1

<210>  1

<211>  116

<212>  PRT

<213>  Artificial

<220>

<221>  DOMAIN

<222>  (1)..(116)

<223>  Antibody-VH-Region from L19

<400>  1

<210>  2

<211>  108

<212>  PRT

<213>  Artificial

<220>

<221>  DOMAIN

<222>  (1)..(108)

<223>  Antibody-VL-Region from L19

<400>  2

<210>  3

<211>  12

<212>  PRT

<213>  Artificial

<220>

<221>  DOMAIN

<222>  (1)..(12)

<223>  Antibody linker region

<400>  3

Gly Asp Gly Ser Ser Gly Gly Ser Gly Gly Ala Ser
1               5                   10

<210>  4

<211>  133

<212>  PRT

<213>  Artificial

<220>

<221>  DOMAIN

<222>  (1)..(133)

<223>  human Interleukin-2

<400>  4

<210>  5

<211>  17

<212>  PRT

<213>  Artificial

<220>

<221>  DOMAIN

<222>  (1)..(17)

<223>  Fusion Protein linker

<400>  5

Glu Phe Ser Ser Ser Ser Gly Ser Ser Ser Ser Gly Ser Ser Ser Ser
1               5                   10                  15

Gly

<210>  6

<211>  155

<212>  PRT

<213>  Artificial

<220>

<221>  DOMAIN

<222>  (1)..(155)

<223>  human TNF alpha

<400>  6

Ser Ser Ser Arg Thr Pro Ser Asp Lys Pro Val Ala His Val Val Ala
1               5                   10                  15

Asn Pro Gln Ala Glu Gly Gln Leu Gln Trp Leu Asn Arg Arg Ala Asn

20                  25                  30

Ala Leu Leu Ala Asn Gly Val Glu Leu Arg Asp Asn Gln Leu Val Val
        35                  40                  45

Pro Ser Glu Gly Leu Tyr Leu Ile Tyr Ser Gln Val Leu Phe Lys Gly
    50                  55                  60

Gln Gly Cys Pro Ser Thr His Val Leu Leu Thr His Thr Ile Ser Arg
65                  70                  75                  80

Ile Ala Val Ser Tyr Gln Thr Lys Val Asn Leu Leu Ser Ala Ile Lys
                85                  90                  95

Ser Pro Cys Gln Arg Glu Thr Pro Glu Gly Ala Glu Ala Lys Pro Trp
            100                 105                 110

Tyr Glu Pro Ile Tyr Leu Gly Gly Val Phe Gln Leu Glu Lys Gly Asp
        115                 120                 125

Arg Leu Ser Ala Glu Ile Asn Arg Pro Asp Tyr Leu Asp Phe Ala Glu
    130                 135                 140

Ser Gly Gln Val Tyr Phe Gly Ile Ile Ala Leu
145                 150                 155

SEQUENCE IDENTIFIER

<110> Schering Aktiengesellschaft

<120> Use of a fusion protein targeting the ED-B fibronectin domain for
treatment of artherosclerosis

<130> 53471AEP

<140>
<141>

<160> 5

<170> PatentIn Ver. 2.1

<210> Sequence Identifier No.1
<211> 116
<212> PRT
<213> Artificial Sequence

<220>
<221> DOMAIN
<222> (1)..(116)
<223> Antibody-VH-Region from L19

<400> 1


  1               5                  10                  15


             20                  25                  30


         35                  40                  45


     50                  55                  60


 65                  70                  75                  80


                 85                  90                  95


            100                 105                 110


        115

<210> 2
<211> 108
<212> PRT
<213> Artificial Sequence

<220>
<221> DOMAIN
<222> (1)..(108)

<223> Antibody-VL-Region from L19

<400> 2

<210> 3
<211> 12
<212> PRT
<213> Artificial Sequence

<220>
<221> DOMAIN
<222> (1)..(12)
<223> Antibody linker region

<400> 3

Gly Asp Gly Ser Ser Gly Gly Ser Gly Gly Ala Ser
1               5                   10

<210> 4
<211> 133
<212> PRT
<213> Antificial Sequence

<220>
<221> DOMAIN
<222> (1)..(133)
<223> human Interleukin-2

<400> 4

<210> 5
<211> 17
<212> PRT
<213> Artificial Sequence

<220>
<221> DOMAIN
<222> (1)..(17)
<223> Fusion Protein linker

<400> 5

Glu Phe Ser Ser Ser Ser Gly Ser Ser Ser Se Gly Ser Ser Ser Ser
 1               5                  10                  15

Gly

<210> 6
<211> 155
<212> PRT
<213> Antificial Sequence

<220>
<221> DOMAIN
<222> (1)..(155)
<223> human TNF alpa

<400> 6
Ser Ser Ser Arg Thr Pro Ser Asp Lys Pro Val Ala His Val Val Ala
                005                 010                 015

Asn Pro Gln Ala Glu Gly Gln Leu Gln Trp Leu Asn Arg Arg Ala Asn
            020                 025                 030

Ala Leu Leu Ala Asn Gly Val Glu Leu Arg Asp Asn Gln Leu Val Val
        035                 040                 045

Pro Ser Glu Gly Leu Tyr Leu Ile Tyr Ser Gln Val Leu Phe Lys Gly
    050                 055                 060

Gln Gly Cys Pro Ser Thr His Val Leu Leu Thr His Thr Ile Ser Arg
065                 070                 075                 080

Ile Ala Val Ser Tyr Gln Thr Lys Val Asn Leu Leu Ser Ala Ile Lys
                085                 090                 095

Ser Pro Cys Gln Arg Glu Thr Pro Glu Gly Ala Glu Ala Lys Pro Trp
            100                 105                 110

Tyr Glu Pro Ile Tyr Leu Gly Gly Val Phe Gln Leu Glu Lys Gly Asp
        115                 120                 125

Arg Leu Ser Ala Glu Ile Asn Arg Pro Asp Tyr Leu Asp Phe Ala Glu
    130                 135                 140

Ser Gly Gln Val Tyr Phe Gly Ile Ile Ala Leu
145                 150                 155